(19) Europäisches Patentamt
European Patent Office
Office européen des brevets

(11) Veröffentlichungsnummer: **0 529 402 A1**

# (12) EUROPÄISCHE PATENTANMELDUNG

(21) Anmeldenummer: **92113669.3**

(22) Anmeldetag: **11.08.92**

(51) Int. Cl.5: **C07D 265/12**, C07D 265/06, C07C 323/52, A01N 43/86

(30) Priorität: **23.08.91 DE 4128031**

(43) Veröffentlichungstag der Anmeldung: **03.03.93 Patentblatt 93/09**

(84) Benannte Vertragsstaaten: **BE CH DE DK ES FR GB IT LI NL**

(71) Anmelder: **BAYER AG**

**W-5090 Leverkusen 1 Bayerwerk(DE)**

(72) Erfinder: **Ooms, Pieter, Dr.**
**Doerperhofstrasse 16**
**W-4150 Krefeld(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen(DE)**
Erfinder: **Schmidt, Robert R., Dr.**
**Im Waldwinkel 110**
**W-5060 Bergisch Gladbach 2(DE)**
Erfinder: **Krauskopf, Birgit, Dr.**
**Kicke 19**
**W-5060 Bergisch Gladbach(DE)**

## (54) N-(3-Alkylthio-phenyl)-oxazindione Herbizide.

(57) Die Erfindung betrifft neue N-(3-Alkylthio-phenyl)-oxazindione der allgemeinen Formel (I)

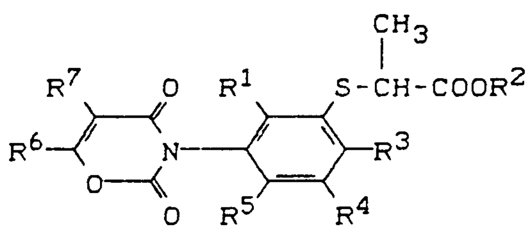

(I)

in welcher

- $R^1$ für Wasserstoff oder Halogen steht,
- $R^2$ für $C_1$-$C_6$-Alkyl, $C_3$-$C_6$-Alkenyl, $C_3$-$C_6$-Alkinyl, $C_3$-$C_6$-Cycloalkyl, $C_5$-$C_6$-Cycloalkenyl, $C_3$-$C_6$-Cycloalkyl-$C_1$-$C_4$-alkyl, $C_5$-$C_6$-Cycloalkenyl-$C_1$-$C_4$-alkyl, $C_1$-$C_4$-Alkoxy-$C_1$-$C_4$-alkyl, $C_1$-$C_4$-Alkylthio-$C_1$-$C_4$-alkyl, Benzyloxy-$C_1$-$C_4$-alkyl, $C_1$-$C_4$-Alkoxy-carbonyl-$C_1$-$C_4$-alkyl, $C_1$-$C_4$-Alkylamino-carbonyl-$C_1$-$C_4$-alkyl oder Di-($C_1$-$C_4$-alkyl)-amino-carbonyl-$C_1$-$C_4$-alkyl steht,
- $R^3$ für Wasserstoff, Halogen, Cyano, Methyl oder Trifluormethyl steht,
- $R^4$ für Wasserstoff oder Halogen steht,
- $R^5$ für Wasserstoff oder Halogen steht,
- $R^6$ für Wasserstoff, Halogen, $C_1$-$C_4$-Alkyl oder $C_1$-$C_4$-Halogenalkyl steht und
- $R^7$ für Wasserstoff, Halogen, $C_1$-$C_4$-Alkyl oder $C_1$-$C_4$-Halogenalkyl steht, oder zusammen mit $R^6$ für $C_3$-$C_5$-Alkandiyl steht,

Verfahren zu ihrer Herstellung und ihre Verwendung als Herbizide, sowie neue Zwischenprodukte.

EP 0 529 402 A1

Die Erfindung betrifft neue N-(3-Alkylthio-phenyl)-oxazindione, Verfahren zu ihrer Herstellung und ihre Verwendung als Herbizide.

Es ist bekannt, daß bestimmte N-Phenyl-substituierte Oxazindione, wie z.B. 3-(2-Fluor-4-chlor-5-chlordifluormethoxy-phenyl)-6,7-dihydro-cyclopent[e]-1,3-oxazin-2,4-(3H,5H)-dion, herbizide Eigenschaften aufweisen (vgl. EP-A 371240). Die Wirkung dieser bekannten Verbindungen ist jedoch nicht in allen Belangen zufriedenstellend.

Es wurden nun neue N-(3-Alkylthio-phenyl)-oxazindione der allgemeinen Formel (I) gefunden,

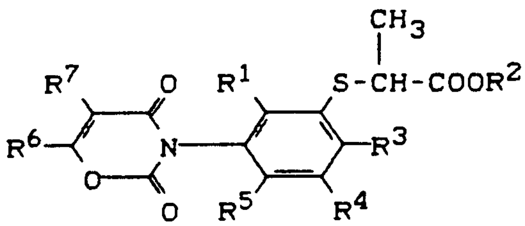

(I)

in welcher

$R^1$ für Wasserstoff oder Halogen steht,

$R^2$ für $C_1$-$C_6$-Alkyl, $C_3$-$C_6$-Alkenyl, $C_3$-$C_6$-Alkinyl, $C_3$-$C_6$-Cycloalkyl, $C_5$-$C_6$-Cycloalkenyl, $C_3$-$C_6$-Cycloalkyl-$C_1$-$C_4$-alkyl, $C_5$-$C_6$-Cycloalkenyl-$C_1$-$C_4$-alkyl, $C_1$-$C_4$-Alkoxy-$C_1$-$C_4$-alkyl, $C_1$-$C_4$-Alkylthio-$C_1$-$C_4$-alkyl, Benzyloxy-$C_1$-$C_4$-alkyl, $C_1$-$C_4$-Alkoxy-carbonyl-$C_1$-$C_4$-alkyl, $C_1$-$C_4$-Alkylamino-carbonyl-$C_1$-$C_4$-alkyl oder Di-($C_1$-$C_4$-alkyl)-amino-carbonyl-$C_1$-$C_4$-alkyl steht,

$R^3$ für Wasserstoff, Halogen, Cyano, Methyl oder Trifluormethyl steht,

$R^4$ für Wasserstoff oder Halogen steht,

$R^5$ für Wasserstoff oder Halogen steht,

$R^6$ für Wasserstoff, Halogen, $C_1$-$C_4$-Alkyl oder $C_1$-$C_4$-Halogenalkyl steht und

$R^7$ für Wasserstoff, Halogen, $C_1$-$C_4$-Alkyl oder $C_1$-$C_4$-Halogenalkyl steht, oder zusammen mit $R^6$ für $C_3$-$C_5$-Alkandiyl steht.

Man erhält die neuen N-(3-Alkylthio-phenyl)-oxazindione der allgemeinen Formel (I), wenn man Arylisocyanate der allgemeinen Formel (II)

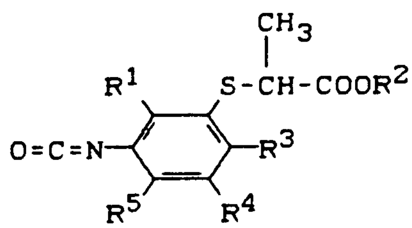

(II)

in welcher

$R^1$, $R^2$, $R^3$, $R^4$ und $R^5$ die oben angegebene Bedeutung haben,

(a) mit 1,3-Dioxin-4-onen der allgemeinen Formel (III)

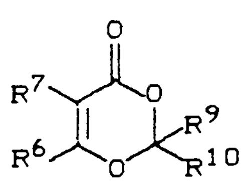

(III)

in welcher

$R^6$ und $R^7$ die oben angegebenen Bedeutungen haben und

$R^9$ und $R^{10}$ für Wasserstoff oder gegebenenfalls substituiertes Alkyl stehen,

gegebenenfalls in Gegenwart eines Verdünnungsmittels umsetzt, oder

(b) mit Dicarbonsäuredichloriden der allgemeinen Formel (IV)

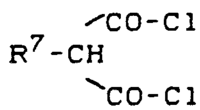

(IV)

in welcher

$R^7$ die oben angegebene Bedeutung hat,

gegebenenfalls in Gegenwart eines Verdünnungsmittels umsetzt, oder

(c) mit 2-Diazo-1,3-diketonen der allgemeinen Formel (V)

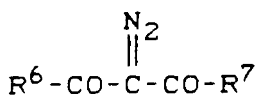

(V)

in welcher

$R^6$ und $R^7$ die oben angegebenen Bedeutungen haben,

gegebenenfalls in Gegenwart eines Verdünnungsmittels umsetzt.

Die neuen N-(3-Alkylthio-phenyl)-oxazindione der allgemeinen Formel (I) zeichnen sich durch starke herbizide Wirksamkeit aus.

Überraschenderweise zeigen die erfindungsgemäßen Verbindungen der Formel (I) erheblich stärkere Herbizidwirkung, als die nach Struktur und Wirkprofil vergleichbare bekannte Verbindung 3-(2-Fluor-4-chlor-5-chlordifluormethoxy-phenyl)-6,7-dihydro-cyclopent[e]-1,3-oxazin-2,4-(3H,5H)-dion.

Die in den Definitionen genannten gesättigten oder ungesättigten Kohlenwasserstoffketten, wie Alkyl, Alkenyl, Alkinyl oder Alkoxy sind jeweils geradkettig, können aber gegebenenfalls auch verzweigt sein.

Halogen steht im allgemeinen für Fluor, Chlor, Brom oder Jod, vorzugsweise für Fluor, Chlor oder Brom.

Die Erfindung betrifft vorzugsweise Verbindungen der Formel (I), in welcher

$R^1$ für Wasserstoff steht,

$R^2$ für Methyl, Ethyl, Propyl, Isopropyl, Butyl, Isobutyl, sec-Butyl, Allyl, Crotonyl, Propargyl, Cyclopentyl, Cyclohexyl, Cyclopentenyl, Cyclohexenyl, Cyclopropylmethyl, Cyclopentylmethyl, Cyclohexylmethyl, Cyclopentenylmethyl, Cyclohexenylmethyl, Methoxyethyl, Ethoxyethyl, Methylthioethyl, Ethylthioethyl, Benzyloxyethyl, Methoxycarbonylmethyl, Ethoxycarbonylmethyl, Methoxycarbonylethyl, Ethoxycarbonylethyl, Methylaminocarbonylmethyl, Ethylaminocarbonylmethyl, Methylaminocarbonylethyl, Ethylaminocarbonylethyl oder Dimethylaminocarbonylmethyl steht,

$R^3$ für Wasserstoff, Chlor, Brom, Cyano, Methyl oder Trifluormethyl steht,

$R^4$ für Wasserstoff steht,

$R^5$ für Wasserstoff oder Fluor steht,

$R^6$ für Wasserstoff, Chlor, Brom, Methyl, Ethyl, Propyl, Isopropyl oder Trifluormethyl steht und

$R^7$ für Wasserstoff, Chlor, Brom, Methyl, Ethyl, Propyl, Isopropyl oder Trifluormethyl steht oder zusammen mit $R^6$ für $C_3$-$C_4$-Alkandiyl (Trimethylen oder Tetramethylen) steht.

Beispiele für die erfindungsgemäßen Verbindungen der Formel (I) sind in der nachstehenden Tabelle 1 aufgeführt.

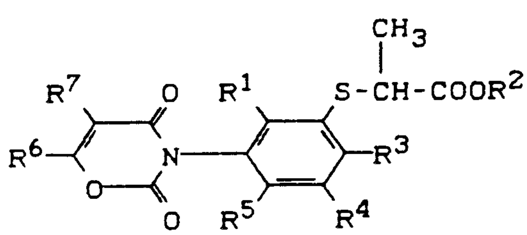

(I)

Tabelle 1: Beispiele für die Verbindungen der Formel (I)

| $R^1$ | $R^2$ | $R^3$ | $R^4$ | $R^5$ | $R^6$ | $R^7$ |
|---|---|---|---|---|---|---|
| H | $CH_3$ | Cl | H | F | $CH_3$ | H |
| H | $C_2H_5$ | Br | H | F | $CH_3$ | $CH_3$ |
| H | $C_3H_7$ | CN | H | H | $CH_3$ | $C_2H_5$ |
| H | $CH_3$ | $CH_3$ | H | H | $CH_3$ | $CH_3$ |
| H | $CH(CH_3)_2$ | Cl | H | F | $-(CH_2)_3-$ | |
| H | $C_4H_9$ | Cl | H | F | $-(CH_2)_4-$ | |
| H | $-CH_2-CH=CH_2$ | Cl | H | F | $CH_3$ | $C_2H_5$ |
| H | $-CH_2-C\equiv CH$ | Cl | H | F | $-(CH_2)_3-$ | |
| H | $-CH_2CH_2OCH_3$ | Cl | H | F | $CH_3$ | $CH_3$ |
| H | $-CH_2CH_2SC_2H_5$ | Cl | H | F | $-(CH_2)_4-$ | |
| H | $-CH_2COOC_2H_5$ | CN | H | H | $-(CH_2)_3-$ | |
| H | $-CH_2CONHC_2H_5$ | Cl | H | F | $-(CH_2)_4-$ | |
| H | $-CH_2CON(CH_3)_2$ | Cl | H | F | $CH_3$ | $CH_3$ |
| H | $CH_2CH(CH_3)_2$ | Cl | H | F | $CH_3$ | $CH_3$ |
| H | $-CH_2$ 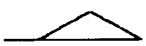 | Cl | H | F | $-(CH_2)_3-$ | |
| H | 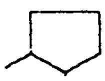 | Cl | H | F | $-(CH_2)_4-$ | |
| H | $-CH_2$ 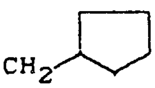 | Cl | H | F | $CH_3$ | $CH_3$ |
| H | 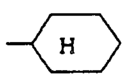 | Cl | H | F | $CH_3$ | $CH_3$ |

Tabelle 1 - Fortsetzung

| $R^1$ | $R^2$ | $R^3$ | $R^4$ | $R^5$ | $R^6$ | $R^7$ |
|---|---|---|---|---|---|---|
| H | $-CH_2CH_2OC_2H_5$ | Cl | H | F | $-(CH_2)_3-$ | |
| H | $-CH_2CH_2SCH_3$ | Cl | H | F | $-(CH_2)_4-$ | |
| H | $-CHC_2H_5$<br>$CH_3$ | Cl | H | F | $-(CH_2)_3-$ | |
| H | $-CHCOOCH_3$<br>$CH_3$ | Cl | H | F | $CH_3$ | $CH_3$ |
| H | $-CHCOOC_2H_5$<br>$CH_3$ | Cl | H | F | $-(CH_2)_3-$ | |

Verwendet man beim erfindungsgemäßen Verfahren (a) beispielsweise A-Chlor-2-fluor-5-(1-methoxycarbonyl-ethylthio)-phenylisocyanat und 2,2,6-Trimethyl-1,3-dioxin-4-on als Ausgangsstoffe, so kann der Reaktionsablauf durch das folgende Formelschema skizziert werden:

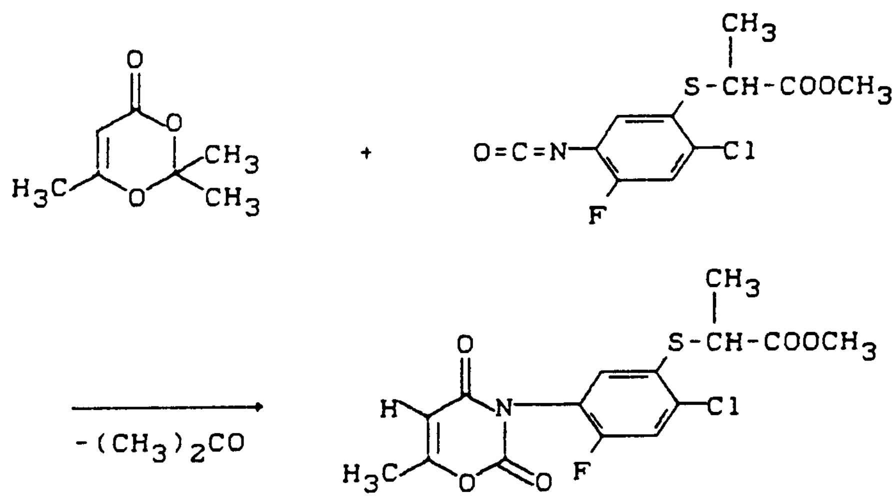


Verwendet man beim erfindungsgemäßen Verfahren (b) beispielsweise Methylmalonsäure-dichlorid und 4-Cyano-3-(1-propoxycarbonyl-ethylthio)-phenylisocyanat als Ausgangsstoffe, so kann der Reaktionsablauf durch das folgende Formelschema skizziert werden:

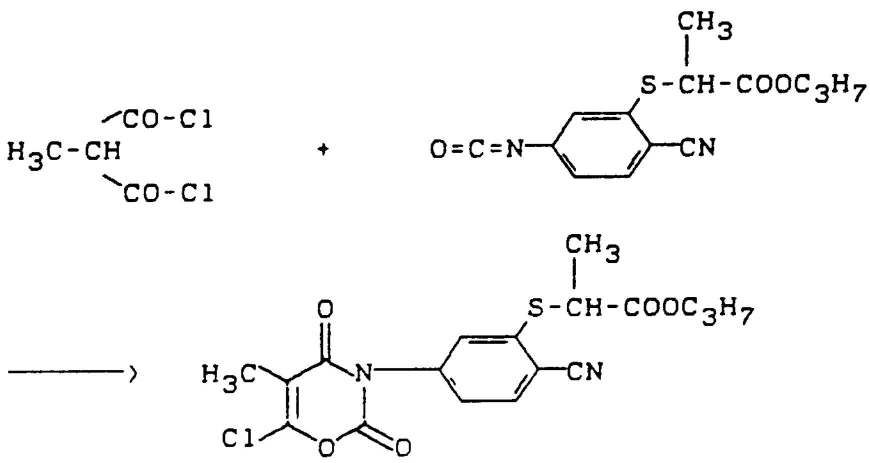

Verwendet man beim erfindungsgemäßen Verfahren (c) beispielsweise 4-Brom-2-fluor-5-(1-ethoxycarbonyl-ethylthio)-phenylisocyanat und 2-Diazo-cyclohexan-1,3-dion als Ausgangsstoffe, so kann der Reaktionsablauf durch das folgende Formelschema skizziert werden:

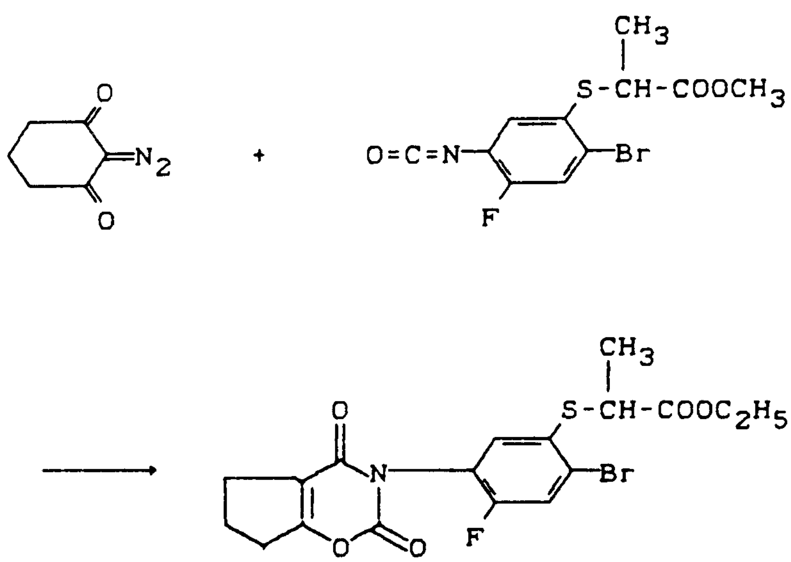

Die bei den erfindungsgemäßen Verfahren (a), (b) und (c) zur Herstellung von Verbindungen der Formel (I) als Ausgangsstoffe zu verwendenden Arylisocyanate sind durch die Formel (II) allgemein definiert.

In Formel (II) haben $R^1$, $R^2$, $R^3$, $R^4$ und $R^5$ vorzugsweise diejenigen Bedeutungen, die bereits oben im Zusammenhang mit der Beschreibung der erfindungsgemäßen Verbindungen der Formel (I) vorzugsweise für $R^1$, $R^2$, $R^3$, $R^4$ und $R^5$ angegeben wurden.

Die Ausgangsstoffe der Formel (II) sind noch nicht aus der Literatur bekannt und sind als neue Stoffe Gegenstand der vorliegenden Patentanmeldung.

Man erhält die neuen Arylisocyanate der Formel (II), wenn man Arylamine der allgemeinen Formel (VI)

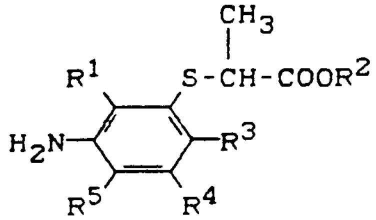

(VI)

in welcher

$R^1$, $R^2$, $R^3$, $R^4$ und $R^5$ die oben angegebene Bedeutung haben,

mit Phosgen, gegebenenfalls in Gegenwart eines Verdünnungsmittels, wie z.B. Toluol oder Chlorbenzol, bei Temperaturen zwischen 0°C und 150°C umsetzt.

Die Arylamine der Formel (VI) sind bekannt und/oder können nach an sich bekannten Verfahren hergestellt werden (vgl. JP-A 63039859 - zitiert in Chem. Abstracts 109: 37735p; JP-A 02191261 - zitiert in Chem. Abstracts 114: 81861d).

Die beim erfindungsgemäßen Verfahren (a) zur Herstellung von Verbindungen der Formel (I) weiter als Ausgangsstoffe zu verwendenden 1,3-Dioxin-4-one sind durch die Formel (III) allgemein definiert.

In Formel (III) haben $R^6$ und $R^7$ vorzugsweise diejenigen Bedeutungen, die bereits oben im Zusammenhang mit der Beschreibung der erfindungsgemäßen Verbindungen der Formel (I) vorzugsweise für $R^6$ und $R^7$ angegeben wurden, und $R^9$ und $R^{10}$ stehen vorzugsweise für Wasserstoff oder $C_1$-$C_6$-Alkyl, insbesondere für Methyl.

Die Ausgangsstoffe der Formel (III) sind bekannt und/oder können nach an sich bekannten Verfahren hergestellt werden (vgl. Chem. Ber. 105 (1972), 137 - 149; J. Am. Chem. Soc. 74 (1952), 6305 - 6306; loc. cit. 75 (1953), 5400 - 5402; DE-OS 19 57 312).

Die beim erfindungsgemäßen Verfahren (b) zur Herstellung von Verbindungen der Formel (I) als Ausgangsstoffe zu verwendenden Dicarbonsäure-dichloride sind durch die Formel (IV) allgemein definiert.

In Formel (IV) hat $R^7$ vorzugsweise diejenige Bedeutung, die bereits oben im Zusammenhang mit der Beschreibung der erfindungsgemäßen Verbindungen der Formel (I) vorzugsweise für $R^7$ angegeben wurde.

Als Beispiele für die Ausgangsstoffe der Formel (IV) seien genannt:
Malonsäure-dichlorid, Methylmalonsäure-dichlorid und Ethylmalonsäure-dichlorid.

Die Dicarbonsäure-dichloride der Formel (IV) sind bekannte organische Synthesechemikalien.

Die beim erfindungsgemäßen Verfahren (c) zur Herstellung von Verbindungen der Formel (I) als Ausgangsstoffe zu verwendenden 2-Diazo-1,3-diketone sind durch die Formel (V) allgemein definiert.

In Formel (V) haben $R^6$ und $R^7$ vorzugsweise diejenigen Bedeutungen, die bereits oben im Zusammenhang mit der Beschreibung der erfindungsgemäßen Verbindungen der Formel (I) vorzugsweise für $R^6$ und $R^7$ angegeben wurden.

Als Beispiele für die Ausgangsstoffe der Formel (V) seien genannt:
2-Diazo-butan-1,3-dion, 3-Diazo-pentan-2,4-dion, 3-Diazo-hexan-2,4-dion, 2-Diazo-cyclopentan-1,3-dion, 2-Diazo-cyclohexan-1,3-dion, 2-Diazo-cylcoheptan-1,3-dion und 2-Diazo-5,5-dimethyl-cyclohexan-1,3-dion.

Die Ausgangsstoffe der Formel (V) sind bekannt und/oder können nach an sich bekannten Verfahren hergestellt werden (vgl. Liebigs Ann. Chem. 687 (1965), 214 - 231; Chem. Ber. 99 (1966), 3128 - 3147; loc cit. 104 (1971), 1942 - 1956).

Das erfindungsgemäße Verfahren (a) zur Herstellung der neuen Verbindungen der Formel (I) wird gegebenenfalls unter Verwendung von Verdünnungsmitteln durchgeführt. Als Verdünnungsmittel kommen vorzugsweise hochsiedende, inerte organische Lösungsmittel in Betracht. Hierzu gehören vor allem, gegebenenfalls halogenierte, Kohlenwasserstoffe, wie Dekalin, Tetralin, Toluol, Xylol, ferner Chlorbenzol und 1,2-Dichlorbenzol sowie Mesitylen.

Die Reaktionstemperaturen können bei dem erfindungsgemäßen Verfahren (a) in einem größeren Bereich variiert werden. Im allgemeinen arbeitet man bei Temperaturen zwischen 80 °C und 250 °C, vorzugsweise bei Temperaturen zwischen 120 °C und 220 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel - gegebenenfalls aber auch ohne Verdünnungsmittel - durchgeführt und das Reaktionsgemisch wird bei der jeweils erforderlichen Temperatur gerührt. Die Aufarbeitung erfolgt bei dem erfindungsgemäßen Verfahren (a) jeweils nach üblichen Methoden (vgl. auch die Herstellungsbeispiele).

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und

Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden gegebenenfalls in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Das erfindungsgemäße Verfahren (c) zur Herstellung der neuen Verbindungen der Formel (I) wird gegebenenfalls unter Verwendung von Verdünnungsmitteln durchgeführt. Als Verdünnungsmittel kommen die gleichen inerten organischen Lösungsmittel vorzugsweise in Betracht, die oben für Verfahren (a) angegeben sind.

Die Reaktionstemperaturen können bei dem erfindungsgemäßen Verfahren (c) in einem größeren Bereich variiert werden. Im allgemeinen arbeitet man bei Temperaturen zwischen 80 °C und 250 °C, vorzugsweise bei Temperaturen zwischen 120 °C und 220 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden gegebenenfalls in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur Bekämpfung von dikotylen Unkräutern in dikotylen Kulturen, wie z.B. in Soja, vor allem im Nachauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,1 g und 5 kg Wirkstoff pro Hektar Bodenfläche, vorzugsweise zwischen 1 g und 2 kg pro ha.

Die Herstellung und die Verwendung der erfindungsgemäßen Wirkstoffe geht aus den nachfolgenden Beispielen hervor.

Herstellungsbeispiele:

Beispiel 1

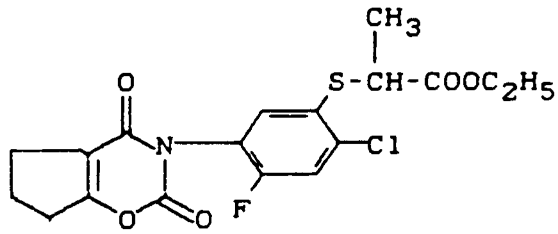

28,3 g (0,17 Mol) 2,2-Dimethyl-4,5,6,7-tetrahydropenta-1,3-dioxin-4-on werden bei 160 °C zu einer Lösung von 42,5 g (0,14 Mol) 4-Chlor-2-fluor-5-(1-ethoxycarbonylethylthio)-phenylisocyanat in 100 ml 1,2-Dichlorbenzol tropfenweise gegeben und das Reaktionsgemisch anschließend noch 2 Stunden bei 160 °C gerührt. Nach Einengen im Vakuum wird der Rückstand durch Säulenchromatographie an Kieselgel gereinigt.

Man erhält 19,3 g (33% der Theorie) 3-(2-Fluor-4-chlor-5-(1-ethoxycarbonyl-ethylthio-phenyl)-6,7-dihydro-cyclopent[e]-1,3-oxazin-2,4(3H,5H)-dion als Öl.

$^1$H-NMR (80 MHz, $CDCl_3$, $\delta$): 7.51 (dd, 1H, J = 8.0 Hz, J = 1 Hz); 7.36 (dd, 1H, J = 9.0 Hz, J' = 1 Hz); 4.09 (dq, 2H, J = 8.0 Hz, J' = 2.0 Hz); 3.89 (dq, 1H, J = 8.0 Hz, J' = 2.0 Hz); 2.87 (t, 2H, J = 8.0 Hz); 2.76 (t, 2H, J = 8.0 Hz); 2.18 (quintett, 2H, J = 8.0 Hz); 1.52 (d, 3H, J = 8.0 Hz); 1.15 (dt, 3H, J = 8.0 Hz, J' = 1.0 Hz)

Beispiel 2

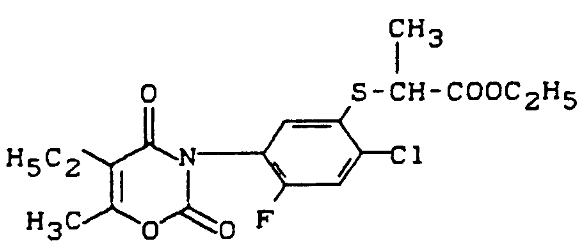

3-(2-Fluor-4-chlor-5-(1-ethoxycarbonyl-ethylthio)-phenyl)-5-ethyl-6-methyl-1,3-oxazin-2,4-dion

$^1$H-HMR (80 MHz, $CDCl_3$, $\delta$): 7.52 (dd, 1H, J = 8.0 Hz, J' = 1 Hz); 7.35 (dd, 1H, J = 9.0 Hz, J' = 1 Hz); 4.09 (dq, 2H, J = 8.0 Hz, J' = 2.0 Hz); 3.88 (q, 1H, J = 8.0 Hz); 2.42 (q, 2H, J = 9.0 Hz); 2.29 (5,3 H); 1.52 (d, 3H, J = 8.0 Hz); 1.29 (t, 3H, J = 8.0 Hz); 1.15 (t, 3H, J = 8.0 Hz)

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegenüber dem Stand der Technik zeigt in diesem Test z.B. die Verbindung gemäß Herstellungsbeispiel 1.

## Patentansprüche

**1.** Substituierte N-(3-Alkylthio-phenyl)-oxazindione der allgemeinen Formel (I)

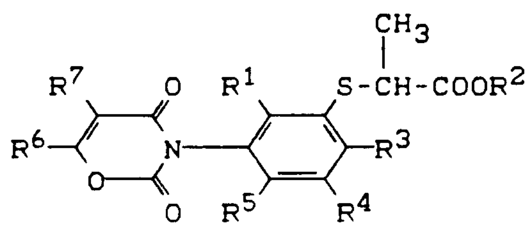

(I)

bei welchen

- $R^1$ für Wasserstoff oder Halogen steht,
- $R^2$ für $C_1$-$C_6$-Alkyl, $C_3$-$C_6$-Alkenyl, $C_3C_6$-Alkinyl, $C_3$-$C_6$-Cycloalkyl, $C_5$-$C_6$-Cycloalkenyl, $C_3$-$C_6$-Cycloalkyl-$C_1$-$C_4$-alkyl, $C_5$-$C_6$-Cycloalkenyl-$C_1$-$C_4$-alkyl, $C_1$-$C_4$-Alkoxy-$C_1$-$C_4$-alkyl, $C_1$-$C_4$-Alkylthio-$C_1$-$C_4$-alkyl, Benzyloxy-$C_1$-$C_4$-alkyl, $C_1$-$C_4$-Alkoxy-carbonyl-$C_1$-$C_4$-alkyl, $C_1$-$C_4$-Alkylamino-carbonyl-$C_1$-$C_4$-alkyl oder Di-($C_1$-$C_4$-alkyl)-amino-carbonyl-$C_1$-$C_4$-alkyl steht,
- $R^3$ für Wasserstoff, Halogen, Cyano, Methyl oder Trifluormethyl steht,
- $R^4$ für Wasserstoff oder Halogen steht,
- $R^5$ für Wasserstoff oder Halogen steht,
- $R^6$ für Wasserstoff, Halogen, $C_1$-$C_4$-Alkyl oder $C_1$-$C_4$-Halogenalkyl steht und
- $R^7$ für Wasserstoff, Halogen, $C_1$-$C_4$-Alkyl oder $C_1$-$C_4$-Halogenalkyl steht, oder zusammen mit $R^6$ für $C_3$-$C_5$-Alkandiyl steht.

**2.** Substituierte N-(3-Alkylthio-phenyl)-oxazindione der allgemeinen Formel (I) gemäß Anspruch 1, bei welchen

- $R^1$ für Wasserstoff steht,
- $R^2$ für Methyl, Ethyl, Propyl, Isopropyl, Butyl, Isobutyl, sec-Butyl, Allyl, Crotonyl, Propargyl, Cyclopentyl, Cyclohexyl, Cyclopentenyl, Cyclohexenyl, Cyclopropylmethyl, Cyclopentylmethyl, Cyclohexylmethyl, Cyclopentenylmethyl, Cyclohexenylmethyl, Methoxyethyl, Ethoxyethyl, Methylthioethyl, Ethylthioethyl, Benzyloxyethyl, Methoxycarbonylmethyl, Ethoxycarbonylmethyl, Methoxycarbonylethyl, Ethoxycarbonylethyl, Methylaminocarbonylmethyl, Ethylaminocarbonylmethyl, Methylaminocarbonylethyl, Ethylaminocarbonylethyl oder Dimethylaminocarbonylmethyl steht,
- $R^3$ für Wasserstoff, Chlor, Brom, Cyano, Methyl oder Trifluormethyl steht,
- $R^4$ für Wasserstoff steht,
- $R^5$ für Wasserstoff oder Fluor steht,

$R^6$ für Wasserstoff, Chlor, Brom, Methyl, Ethyl, Propyl, Isopropyl oder Trifluormethyl steht und

$R^7$ für Wasserstoff, Chlor, Brom, Methyl, Ethyl, Propyl, Isopropyl oder Trifluormethyl steht oder zusammen mit $R^6$ für $C_3$-$C_4$-Alkandiyl (Trimethylen oder Tetramethylen) steht.

**3.** Verfahren zur Herstellung von substituierten N-(3-Alkylthio-phenyl)-oxazindionen der allgemeinen Formel (I) gemäß Anspruch 1

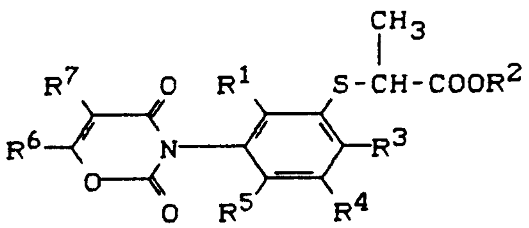

(I)

in welcher

$R^1$ für Wasserstoff oder Halogen steht,

$R^2$ für $C_1$-$C_6$-Alkyl, $C_3$-$C_6$-Alkenyl, $C_3$-$C_6$-Alkinyl, $C_3$-$C_6$-Cycloalkyl, $C_5$-$C_6$-Cycloalkenyl, $C_3$-$C_6$-Cycloalkyl-$C_1$-$C_4$-alkyl, $C_5$-$C_6$-Cycloalkenyl-$C_1$-$C_4$-alkyl, $C_1$-$C_4$-Alkoxy-$C_1$-$C_4$-alkyl, $C_1$-$C_4$-Alkylthio-$C_1$-$C_4$-alkyl, Benzyloxy-$C_1$-$C_4$-alkyl, $C_1$-$C_4$-Alkoxy-carbonyl-$C_1$-$C_4$-alkyl, $C_1$-$C_4$-Alkylamino-carbonyl-$C_1$-$C_4$-alkyl oder Di-($C_1$-$C_4$-alkyl)-amino-carbonyl-$C_1$-$C_4$-alkyl steht,

$R^3$ für Wasserstoff, Halogen, Cyano, Methyl oder Trifluormethyl steht,

$R^4$ für Wasserstoff oder Halogen steht,

$R^5$ für Wasserstoff oder Halogen steht,

$R^6$ für Wasserstoff, Halogen, $C_1$-$C_4$-Alkyl oder $C_1$-$C_4$-Halogenalkyl steht und

$R^7$ für Wasserstoff, Halogen, $C_1$-$C_4$-Alkyl oder $C_1$-$C_4$-Halogenalkyl steht, oder zusammen mit $R^6$ für $C_3$-$C_5$-Alkandiyl steht,

dadurch gekennzeichnet, daß man

Arylisocyanate der allgemeinen Formel (II)

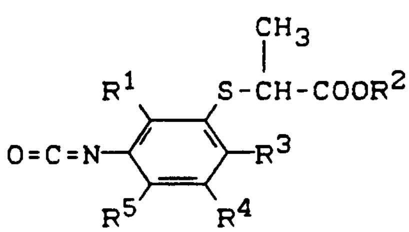

(II)

in welcher

$R^1$, $R^2$, $R^3$, $R^4$ und $R^5$ die oben angegebenen Bedeutungen haben,

(a) mit 1,3-Dioxin-4-onen der allgemeinen Formel (III)

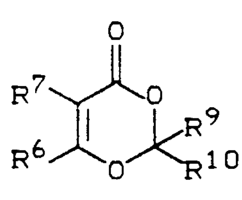

(III)

in welcher

$R^6$ und $R^7$ die oben angegebenen Bedeutungen haben und

$R^9$ und $R^{10}$ für Wasserstoff oder gegebenenfalls substituiertes Alkyl stehen,

gegebenenfalls in Gegenwart eines Verdünnungsmittels umsetzt, oder

(b) mit Dicarbonsäuredichloriden der allgemeinen Formel (IV)

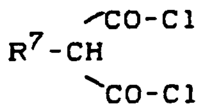 (IV)

in welcher

$R^7$ die oben angegebene Bedeutung hat,

gegebenenfalls in Gegenwart eines Verdünnungsmittels umsetzt, oder

(c) mit 2-Diazo-1,3-diketonen der allgemeinen Formel (V)

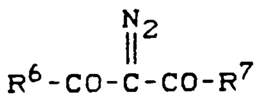 (V)

in welcher

$R^6$ und $R^7$ die oben angegebenen Bedeutungen haben,

gegebenenfalls in Gegenwart eines Verdünnungsmittels umsetzt.

**4.** Herbizide Mittel, gekennzeichnet durch einen Gehalt an mindestens einem substituierten N-(3-Alkylthio-phenyl)-oxazindion der Formel (I) gemäß den Ansprüchen 1 bis 3.

**5.** Verfahren zur Bekämpfung von unerwünschten Pflanzen, dadurch gekennzeichnet, daß man substituierte N-(3-Alkylthiophenyl)-oxazindione der Formel (I) gemäß den Ansprüchen 1 bis 3 auf unerwünschte Pflanzen und/oder ihren Lebensraum einwirken läßt.

**6.** Verwendung von substituierten N-(3-Alkylthiophenyl)-oxazindionen der Formel (I) gemäß den Ansprüchen 1 bis 3 zur Bekämpfung von unerwünschten Pflanzen.

**7.** Verfahren zur Herstellung von herbiziden Mitteln, dadurch gekennzeichnet, daß man substituierte N-(3-Alkylthio-phenyl)-oxazindione der Formel (I) gemäß den Ansprüchen 1 bis 3 mit Streckmitteln und/oder oberflächenaktiven Substanzen vermischt.

**8.** Arylisocyanate der allgemeinen Formel (II)

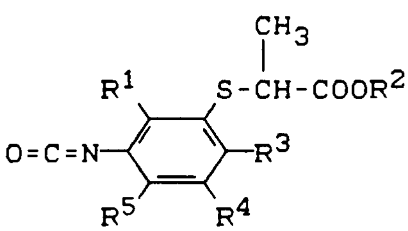 (II)

in welcher

$R^1$ für Wasserstoff oder Halogen steht,

$R^2$ für $C_1$-$C_6$-Alkyl, $C_3$-$C_6$-Alkenyl, $C_3$-$C_6$-Alkinyl, $C_3$- $C_6$-Cycloalkyl, $C_5$-$C_6$-Cycloalkenyl, $C_3$-$C_6$-Cycloalkyl-$C_1$-$C_4$-alkyl, $C_5$-$C_6$-Cycloalkenyl-$C_1$-$C_4$-alkyl, $C_1$-$C_4$-Alkoxy-$C_1$-$C_4$-alkyl, $C_1$-$C_4$-Alkylthio-$C_1$-$C_4$-alkyl, Benzyloxy-$C_1$-$C_4$-alkyl, $C_1$-$C_4$-Alkoxy-carbonyl-$C_1$-$C_4$-alkyl, $C_1$-$C_4$-Alkylamino-carbonyl-$C_1$-$C_4$-alkyl oder Di-($C_1$-$C_4$-alkyl)-amino-carbonyl-$C_1$-$C_4$-alkyl steht,

$R^3$ für Wasserstoff, Halogen, Cyano, Methyl oder Trifluormethyl steht,

$R^4$ für Wasserstoff oder Halogen steht,

$R^5$ für Wasserstoff oder Halogen steht.

Europäisches Patentamt

# EUROPÄISCHER RECHERCHENBERICHT

Nummer der Anmeldung

EP 92 11 3669

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,Y | EP-A-0 371 240 (BAYER AG)<br>* Seite 3, Formel 2; Seiten 12-15, 18, 19, 29, 39 (Zeilen 37-52), 56 (Beispiele 69,70) und Ansprüche. * | 1-8 | C07D265/12<br>C07D265/06<br>C07C323/52<br>A01N43/86 |
| Y | EP-A-0 416 361 (BAYER AG)<br>* Seite 6, Formel 7; Seiten 10-17, Tabelle 1; Seite 28, Beispiel 8 und Ansprüche. * | 1-8 | |
| A | EP-A-0 126 419 (SUMITOMO CHEMICAL CO. LTD.)<br>* Ansprüche * | 1,3,4 | |
| A | EP-A-0 260 228 (CIBA-GEIGY AG)<br>* Ansprüche * | 1,3,4 | |

RECHERCHIERTE SACHGEBIETE (Int. Cl.5 )

C07D
C07C
A01N

Der vorliegende Recherchenbericht wurde für alle Patentansprüche erstellt

| Recherchenort | Abschlußdatum der Recherche | Prüfer |
|---|---|---|
| DEN HAAG | 10 NOVEMBER 1992 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)